# FASCICULE DE BREVET EUROPEEN

(11) **EP 1 063 220 B1**
(45) Date de publication et mention de la délivrance du brevet: **03.09.2003**
(21) Numéro de dépôt: 00401753.9
(22) Date de dépôt: 20.06.2000
(51) Int. Cl.: C07C 45/67, C07C 45/82, C07C 49/603

(54) **Procédé continu de fabrication de 3,5,5-triméthylcyclohexa-3-en-1-one(beta-isophorone)**
Kontinuierliches Verfahren zur Herstellung von 3,5,5-Trimethylcyclohexa-3-en-1-on(beta-Isophoron)
Continuous process for the preparation of 3,5,5-trimethylcyclohexa-3-en-1-one(beta-isophorone)

(30) Priorité: 22.06.1999 FR 9907919
(43) Date de publication de la demande: 27.12.2000
(73) Titulaire: Atofina, 92800 Puteaux (FR)
(72) Inventeur: Mongenet, François, 38200 Chuzelles (FR); Teissier, Rémy, 69340 Francheville (FR)

(56) Documents cités:
- EP-A- 0 832 871
- EP-A- 0 842 918
- FR-A- 2 253 730

## Description

L'invention concerne un procédé continu de fabrication de 3,5,5-triméthylcyclohexa-3-en-1-one, ci-après β-isophorone par isomérisation du 3,5,5 -triméthylcyclohexa -2 -en -1-one, ci-après α-isophorone en phase liquide en présence d'un catalyseur homogène.

La β-isophorone est un intermédiaire de synthèse pour la fabrication de carotinoïdes, de vitamines telles que la vitamine E et de produits pharmaceutiques.

Elle intervient également directement dans des synthèses de parfums et produits naturels comme l'astaxanthine et l'acide abscisine et dérivés.

La β-isophorone est un isomère de l'α-isophorone obtenue par trimérisation de l'acétone en milieu alcalin qui se distingue de celle-ci par la position de la double liaison : la double liaison n'est plus conjuguée au carbonyle comme représenté ci-après :

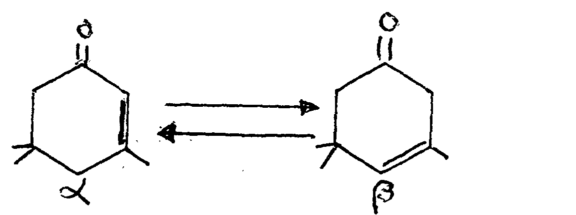

L'isomérisation de l'α-isophorone en β-isophorone est une réaction équilibrée de déconjugaison de la double liaison et du carbonyle et pour cette raison, l'équilibre thermodynamique se situe du côté de l'α-isophorone.

De nombreux procédés d'isomérisation de l'α-isophorone en β-isophorone ont été décrits mais présentent de nombreux inconvénients, tels qu'une consommation élevée de produits chimiques (catalyseurs notamment), rendements médiocres, formation de produits de condensations de l'α-isophorone (produits lourds) qui entraîne une élévation de la température du milieu réactionnel accélérant ainsi la formation de produits lourds qui déstabilisent le système.

Dans le brevet EP 832 871, exemple 7, il est décrit un procédé de préparation de β-isophorone par isomérisation catalytique de l'α-isophorone qui consiste dans un premier temps à extraire, en continu, du milieu réactionnel par distillation un mélange primaire contenant de 20 à 22 % de β-isophorone puis à isoler de ce mélange par distillation une β-isophorone avec une pureté égale à environ 98 %. On constate dans cette façon de procéder que l'on recueille dans l'évaporateur à recirculation un mélange constitué d'environ 90 % d'α-isophorone et 10 % de lourds. Compte tenu de la durée de fonctionnement (environ 15 heures) et de la quantité de produit recueilli dans ledit évaporateur (615 g), cela correspond à une production horaire de produits lourds d'environ 4 g/h.

En outre, on utilise une quantité pondérale importante de catalyseur : 0,6 % par rapport à l'α-isophorone mis en oeuvre.

Si on considère la thermodynamique de la réaction, on constate que la température à laquelle est conduite l'isomérisation fixe la concentration en β-isophorone à l'équilibre : plus la température est haute, plus la concentration à l'équilibre en β-isophorone augmente. Comme la β-isophorone est plus volatile que l'α-isophorone, l'équilibre peut être déplacé par distillation du produit le plus volatile. En outre, la β-isophorone, par chauffage, à une température supérieure à 150°C, se rétroisomérise en α-isophorone, même en absence de catalyseur : la cinétique de cette rétroisomérisation thermique est contrôlée par le niveau de température.

D'autre part, pour conduire l'isomérisation, il faut passer par l'intermédiaire énol ou énolate qui sont aussi les intermédiaires de la réaction d'aldolisation / crotonisation qui conduit à la formation de polycondensats de l'isophorone qui constituent des produits lourds dont la formation est à proscrire puisqu'ils représentent une perte d'isophorone dans un procédé de fabrication de la β-isophorone. La formation des lourds est favorisée d'une part par la concentration en catalyseur et d'autre part par la température.

Ainsi, l'utilisation d'une catalyse hétérogène apparaît comme peu favorable car elle peut augmenter la concentration de produits lourds à la surface du catalyseur solide pouvant désactiver ledit catalyseur.

On a maintenant trouvé un procédé continu de fabrication de 3,5,5-triméthylcyclohexa-3 -en-1 -one (β-isophorone) par isomérisation en catalyse homogène du 3,5,5-triméthylcyclohexa-2-en- 1-one (α-isophorone), obtenue par trimérisation en milieu alcalin de l'acétone, ledit procédé étant caractérisé en ce que l'on effectue les étapes suivantes :
a) on introduit en continu dans une zone réactionnelle l'α-isophorone et une solution d'un hydroxyde alcalin,
b) on porte le milieu réactionnel au reflux à une température allant de 150°C à 216°C et de préférence, à une température allant de 190°C à 216°C, et sous une pression P1 comprise entre 150m bar et la pression atmosphérique,
c) on élimine en continu, simultanément, du milieu réactionnel :
   1/ la β-isophorone de la phase vapeur, par distillation sous une pression P2 inférieure à P1 et comprise entre 50 mbar at 150 mbar et à une température égale ou inférieure à 150 °C,
   2/ des produits lourds, par soutirage, de façon à ce que leur teneur pondérale soit au plus égale à 7 % dans le milieu réactionnel, et
d) on ramène en continu les condensais de distillation à la zone réactionnelle.
et en ce que l'on utilise une quantité pondérale d'hydroxyde alcalin allant de 0,01 % à 0,03% par rapport à l'α-isophorone mise en oeuvre.

Selon la présente invention, la pression P2, inférieure à P1 est, de préférence, comprise entre 50 mbar et 100 mbar.

Selon la présente invention, l'hydroxyde alcalin est NaOH ou KOH. On préfère utiliser KOH. Cet hydroxyde alcalin peut être solubilisé 20 dans de l'eau ou un alcool aliphatique de bas poids moléculaire tel que le méthanol, l'éthanol, le propanol, l'isopropanol. On utilise généralement le méthanol ou l'éthanol.

Selon la présente invention, on utilise une quantité pondérale d'hydroxyde alcalin allant de 0,01% à 0,03 % par rapport à l'α-isophorone introduite dans la zone réactionnelle, et, de preference, on utilisera une quantité pondérale d'hydroxyde alcalin allant de 0,010 % à 0,020 %.

Le taux de reflux de la distillation de la fraction contenant la β-isophorone, paramètre réglant la pureté de cette fraction, est choisi de façon à obtenir une productivité élevée en β-isophorone, c'est-à-dire minimiser la rétrogradation thermique, pour une pureté en β-isophorone maximale.

Selon l'invention, on extrait en continu un mélange contenant un pourcentage pondéral de lourds au plus égal à 7 %. Le reste étant un mélange d'α- et de β-isophorone. Ces lourds sont constitués essentiellement de dimères et trimères de l'isophorone et également de résidus catalytiques. Ces lourds peuvent être avantageusement introduits dans une chaîne de fabrication d'α-isophorone par catalyse basique, après la réaction de trimérisation de l'acétone et avant la neutralisation du catalyseur basique.

Le procédé continu selon la présente invention, caractérisé par un soutirage des produits lourds en continu, présente l'avantage en limitant la concentration desdits produits lourds dans le bouilleur, de stabiliser la température de l'isomérisation.

Un autre avantage du procédé de l'invention est qu'il n'est pas nécessaire d'effectuer des opérations coûteuses de traitement desdits produits lourds puisqu'ils peuvent être recyclés dans une unité de fabrication d'α-isophorone.

Le procédé de l'invention présente également l'avantage de consommer de faibles quantités de charge catalytique.

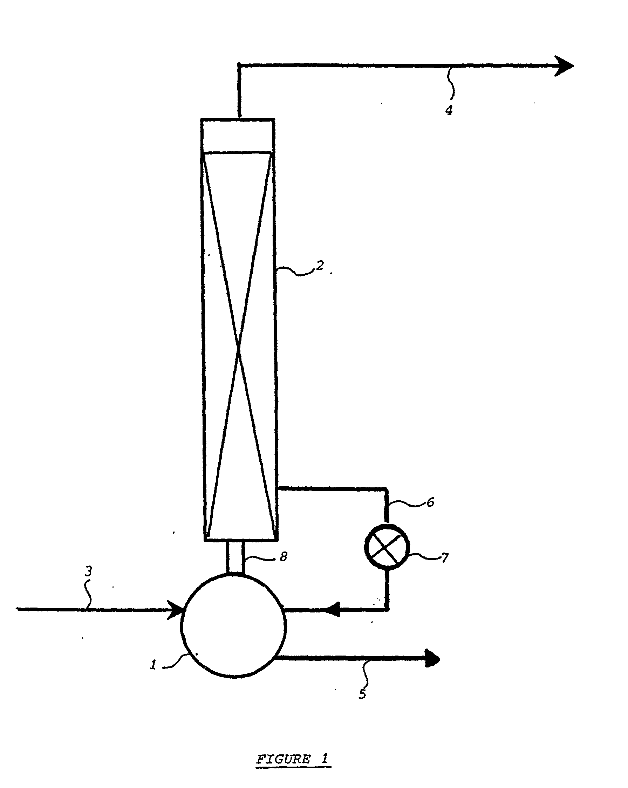

La mise en oeuvre du procédé peut être réalisé dans un appareillage tel que schématisé sur la figure 1 qui est constitué par :
- un bouilleur (1) dans lequel s'effectue la réaction d'isomérisation de l'α-isophorone en β-isophorone surmonté d'une colonne adiabatique (2) pouvant contenir un garnissage tel que le Sulzer EX20 et munie d'une tête de distillation comprenant un timer permettant de régler le taux de reflux ; le taux de reflux R étant défini comme étant le rapport de la quantité totale de condensat obtenue en tête de distillation sur la quantité de produit extrait.

Le bouilleur (1) est muni de moyens de chauffage non représentés sur la figure 1.

L'alimentation du bouilleur (1) est réalisé par la voie (3), la β-isophorone est récupérée par la voie (4) et les produits lourds extraits du bouilleur sont soutirés par la voie (5).

Les condensats sont prélevés en bas de la colonne (2) et ramenés au bouilleur (1) par la voie (6) munie d'une pompe (7).

Un dispositif de détente (8) est intercalé entre le bouilleur (1) et la colonne (2).

Les dimensions de la colonne et du bouilleur sont calculées en fonction de la quantité d'α-isophorone à isomériser et de la productivité désirée et font partie des connaissances générales de l'homme du métier.

A titre d'illustration de la présente invention, il a été réalisé, dans un appareillage de laboratoire, l'essai ci-après.

Un bouilleur constitué par un réacteur en verre de 1 litre contient 300 grammes d'un mélange constitué d'α-isophorone contenant 0,02 % en poids de KOH (exprimé en KOH pur) préalablement dissous dans du méthanol à une concentration pondérale de KOH égale à environ 20 %.

Ce mélange est chauffé à 196°C sous une pression de 600 mbar. Les vapeurs sont injectées en continu dans une colonne adiabatique contenant un garnissage structué Sulzer EX20 en inox 316L sous une pression de 100 mbar. La température en tête de colonne se stabilise à 110°C-111°C.

On extrait, pour un taux de reflux R de 60 une β-isophorone de pureté, définie comme étant le rapport β-isophorone / α-isophorone, supérieure à 99 %.

Le débit d'extraction se stabilise à 4 g/heure.

Les condensais de distillation sont envoyés au bouilleur à l'aide d'une pompe.

L'essai dure 10 heures.

On extrait en continu les lourds.

On compense l'extraction desdits lourds et la production de β-isophorone par introduction d'α-isophorone avec le catalyseur.

## Revendications

1. Procédé continu de fabrication de 3,5,5-triméthylcyclohexa-3-en-1-one(β-isophorone) par isomérisation en catalyse homogène du 3,5,5 -triméthylcyclohexa-2 -en -1 -one(α-isophorone) obtenue par trimérisation en milieu alcalin de l'acétone, ledit procédé étant **caractérisé en ce que** l'on effectue les étapes suivantes :
a) on introduit en continu dans une zone réactionnelle l'α-isophorone et une solution d'un hydroxyde alcalin,
b) on porte le milieu réactionnel au reflux à une température allant de 150°C à 216°C et, sous une pression P1 comprise entre 150 mbar et la pression atmosphérique,
c) on élimine en continu, simultanément du milieu réactionnel :
1/ la β-isophorone de la phase vapeur, par distillation sous une pression P2 inférieure à P1 et comprise entre 50 mbar et 150 mbar et à une température égale ou inférieure à 150°C,
2/ des produits lourds, par soutirage, de façon à ce que leur teneur pondérale soit au plus égale à 7 % dans le milieu réactionnel, et
20 d) on ramène en continu les condensats de distillation à la zone réactionnelle, et **en ce que** l'on utilise une quantité pondérale d'hydroxyde alcalin allant de 0,05% à 0,03% par rapport à l'α-isophorome mise en oeuvre.

2. Procédé selon la revendication 1, **caractérisé en ce que** la pression P2 est comprise entre 50 mbar et 100 mbar.

3. Procédé selon la revendication 1, **caractérisée en ce que** dans l'étape b), le milieu réactionnel est porté à une température allant de 190°C à 216°C.

4. Procédé selon l'une quelconque des revendications 1 à 3, **caractérisé en ce que** l'hydroxyde alcalin est KOH.

5. Procédé selon l'une des revendications 1 ou 4, **caractérisé en ce que** l'hydroxyde alcalin est solubilisé dans un alcool aliphatique de bas poids moléculaire choisi parmi le méthanol, l'éthnol le propanol ou l'isopropanol.

6. Procédé selon la revendication 5, **caractérisé en ce que** l'alcool aliphatique de bas poids moléculaire est le méthanol ou l'éthanol.

7. Procédé selon l'une quelconque des revendications 1 à 6, **caractérisé en ce que** l'on utilise une quantité pondérale d'hydroxyde alcalin allant de 0,010 % à 0,020 % par rapport à l'α-isophorone mise en oeuvre.

8. Procédé selon la revendication 1, **caractérisé en ce que** les produits lourds, soutirés du réacteurs d'isomérisation, étape c) 2/, sont recyclés dans une chaîne de fabrication d'α-isophorone.

9. Procédé selon la revendication 8, **caractérisé en ce que** les produits lourds sont introduits dans une chaîne de fabrication d'α-isophorone après la réaction de trimérisation de l'acétone et avant la neutralisation du catalyseur basique.

## Patentansprüche

1. Kontinuierliches Verfahren zur Herstellung von 3,5,5-Trimethylcyclohex-3-en-1-on (β-Isophoron) durch Isomerisierung in homogener Katalyse von durch Trimerisierung im alkalischen Milieu von Aceton erhaltenen 3,5,5-Trimethylcyclohex-2-en-1-on (α-Isophoron), **dadurch gekennzeichnet, daß** die folgenden Schritte durchgeführt werden:
a) kontinuierliches Einbringen von α-Isophoron und einer Lösung eines alkalischen Hydroxids in eine Reaktionszone,
b) Erhitzen des Reaktionsmilieus unter Rückfluß auf eine Temperatur von 150 °C bis 216 °C unter einem Druck P1 zwischen 150 mbar und Atmosphärendruck,
c) kontinuierliches und gleichzeitiges Entfernen aus dem Reaktionsmilieu von:
1/ β-Isophoron aus der Gasphase durch Destillation unter einem Druck P2, der kleiner als P1 ist und zwischen 50 mbar und 150 mbar liegt, und bei einer Temperatur gleich oder kleiner als 150 °C,
2/ schweren Produkten durch Abziehen, so daß ihr Gewichtsanteil im Reaktionsmilieu höchstens gleich 7 % beträgt, und
d) kontinuierliche Rückrührung der Destillationskondensate in die Reaktionszone und Verwendung einer Gewichtsmenge von alkalischem Hydroxid von 0,01 % bis 0,03 % in bezug auf das eingesetzte α-Isophoron.

2. Verfahren nach Anspruch 1, **dadurch gekennzeichnet, daß** der Druck P2 zwischen 50 mbar und 100 mbar liegt.

3. Verfahren nach Anspruch 1, **dadurch gekennzeichnet, daß** in Schritt b) das Reaktionsmilieu auf eine Temperatur von 190 °C bis 216 °C erhitzt wird.

4. Verfahren nach einem der Ansprüche 1 bis 3, **dadurch gekennzeichnet, daß** das alkalische Hydroxid KOH ist.

5. Verfahren nach einem der Ansprüche 1 oder 4, **dadurch gekennzeichnet, daß** das alkalische Hydroxid in einem aliphatischen Alkohol mit geringem Molekulargewicht gelöst wird, der ausgewählt ist aus Methanol, Ethanol, Propanol oder Isopropanol.

6. Verfahren nach Anspruch 5, **dadurch gekennzeichnet, daß** der aliphatische Alkohol mit geringem Molekulargewicht Methanol oder Ethanol ist.

7. Verfahren nach einem der Ansprüche 1 bis 6, **dadurch gekennzeichnet, daß** eine Gewichtsmenge von alkalischem Hydroxid von 0,010 % bis 0,020 % in bezug auf das eingesetzte α-Isophoron verwendet wird.

8. Verfahren nach Anspruch 1, **dadurch gekennzeichnet, daß** die schweren Produkte, die aus den Isomerisierungsreaktoren in Schritt c) 2/ abgezogen werden, in eine Produktionskette für a-Isophoron zurückgerührt werden.

9. Verfahren nach Anspruch 8, **dadurch gekennzeichnet, daß** die schweren Produkte in eine Produktionskette für α-Isophoron nach der Trimerisierungsreaktion des Acetons und vor der Neutralisierung des basischen Katalysators eingebracht werden.

## Claims

1. Continuous process for the manufacture of 3,5,5-trimethylcyclohexa-3-en-1-one (β-isophorone) by isomerization under homogeneous catalysis of 3,5,5-trimethylcyclohexa-2-en-1-one (α-isophorone), obtained by trimerization of acetone in alkaline medium, the said process being **characterized in that** the following stages are carried out:
a) α-isophorone and a solution of an alkaline hydroxide are continuously introduced into a reaction region,
b) the reaction mixture is brought to reflux at a temperature ranging from 150°C to 216°C and under a pressure P1 of between 150 mbar and atmospheric pressure,
c) the following are continuously and simultaneously removed from the reaction mixture:
1) the β-isophorone of the vapour phase, by distillation under a pressure P2 which is less than P1 and which is between 50 mbar and 150 mbar and at a temperature equal to or less than 150°C,
2) heavy products, by drawing off, so that their content by weight is at most equal to 7% in the reaction mixture, and
d) the distillation condensates are continuously returned to the reaction region,
and **in that** use is made of an amount by weight of alkaline hydroxide ranging from 0.01% to 0.03% with respect to the α-isophorone employed.

2. Process according to Claim 1, **characterised in that** the pressure P2 is between 50 mbar and 100 mbar.

3. Process according to Claim 1, **characterized in that**, in stage b), the reaction mixture is brought to a temperature ranging from 190°C to 216°C.

4. Process according to any one of Claims 1 to 3, **characterized in that** the alkaline hydroxide is KOH.

5. Process according to one of Claims 1 to 4, **characterized in that** the alkaline hydroxide is dissolved in an aliphatic alcohol of low molecular weight chosen from methanol, ethanol, propanol or isopropanol.

6. Process according to Claim 5, **characterized in that** the aliphatic alcohol of low molecular weight is methanol or ethanol.

7. Process according to any one of Claims 1 to 6, **characterized in that** use is made of an amount by weight of alkaline hydroxide ranging from 0.010% to 0.020% with respect to the α-isophorone employed.

8. Process according to Claim 1, **characterized in that** the heavy products drawn off from the isomerization reactor, stage c) 2), are recycled in a line for the manufacture of α-isophorone.

9. Process according to Claim 8, **characterized in that** the heavy products are introduced into a line for the manufacture of α-isophorone after the trimerization reaction of the acetone and before the neutralization of the basic catalyst.
